# EUROPEAN PATENT APPLICATION

(11) **EP 4 527 913 A1**
(43) Date of publication of application: **26.03.2025**
(21) Application number: 22942652.3
(22) Date of filing: 18.05.2022
(51) Int. Cl.: C12M 1/00, C12M 1/34, C12N 5/10, C12N 15/11, C12N 15/113, C12Q 1/06, C12Q 1/6813, C12Q 1/6851

(54) **PREDICTION DEVICE, AUTOMATIC CULTURE DEVICE SYSTEM, PREDICTION METHOD, AND KIT**

(71) Applicant: Hitachi, Ltd., Tokyo 100-8280 (JP)
(72) Inventor: SAITO Hikaru, Tokyo 100-8280 (JP); HIRAI Kakuro, Tokyo 100-8280 (JP); KATO Midori, Tokyo 100-8280 (JP); KIYAMA Masaharu, Tokyo 100-8280 (JP); HANZAWA Hiroko, Tokyo 100-8280 (JP); TAKEDA Shizu, Tokyo 100-8280 (JP)
(74) Representative: Strehl & Partner mbB
(86) International application number: PCT/JP2022/020605
(87) International publication number: WO 2023/223447

(57) **Abstract**

The present invention provides a prediction device, a prediction method, and a kit for predicting a future differentiation level of a cultured cell before start of differentiation induction, based on a content of miRNA in an exosome in the cultured cell, and an automatic cell culture system.

## Description

### Technical Field

The present invention relates to a prediction device, a prediction method, and a kit for predicting a differentiation level of a cultured cell, and an automatic cell culture system.

### Background Art

In order to stabilize and improve the quality of cell production using an automatic cell culture device, it is important to monitor a culture state of cells from the outside without extracting the cells and to perform control in accordance with the culture state. For example, pluripotent cells can be differentiated into various cells by a differentiation induction process, and thus, a target differentiated cell is produced using the pluripotent cells. However, differentiation does not proceed well due to various factors, and a ratio (differentiation rate) of differentiated cells in a finally obtained cell population may be low.

PTL 1 discloses that differentiation from iPS cells to dopamine neural progenitor cells is induced, there is a correlation between the expression level of a marker of dopamine neural progenitor cells in a culture supernatant and the number of culture days, and evaluating the differentiation level of cells during differentiation induction makes it possible to predict the differentiation rate of terminally differentiated cells.

### Citation List

### Patent Literature

PTL 1: JP 2021-153504 A (US2021/0301248A1)

### Summary of Invention

### Technical Problem

In PTL 1, the differentiation level of cells during differentiation induction is evaluated, and a certain culture time has been required to evaluate the differentiation level. Accordingly, it has been necessary to culture cells having a low differentiation level for a certain period of time during differentiation induction.

Thus, an object of the present invention is to provide a prediction device, a prediction method, and a kit for predicting a future differentiation level of a cultured cell before start of differentiation induction, and an automatic cell culture system.

### Solution to Problem

One aspect of the present invention relates to a prediction device for predicting a differentiation level of a cultured cell, and the prediction device includes an analysis device that is configured to predict the differentiation level of the cultured cell based on a content of miRNA in an exosome in a culture supernatant before differentiation induction of the cultured cell.

Another aspect of the present invention relates to an automatic cell culture system, and the automatic cell culture system includes: a culture device including: a cell culture container for culturing a cell; and a drainage container for discarding a culture supernatant of the cultured cell; and a prediction device including: a measurement device that is configured to measure a content of miRNA in an exosome in the culture supernatant before differentiation induction of the cultured cell; and an analysis device that is configured to predict a differentiation level of the cultured cell from the content of the miRNA.

Still another aspect of the present invention relates to a prediction method, and the method includes: measuring a content of miRNA in an exosome in a culture supernatant before differentiation induction of the cultured cell; and predicting the differentiation level of the cultured cell based on the content.

Yet still another aspect of the present invention relates to a kit for predicting a differentiation level of a cultured cell, and the kit includes a means for measuring miRNA in an exosome derived from the cultured cell, in which the differentiation level of the cultured cell is predicted by a method including the steps of: measuring a content of miRNA in an exosome in a culture supernatant before differentiation induction of the cultured cell; and predicting the differentiation level of the cultured cell based on the content.

### Advantageous Effects of Invention

According to the present invention, it is possible to provide a prediction device, a prediction method, and a kit for predicting a future differentiation level of a cultured cell before start of differentiation induction, and an automatic cell culture system. Problems, configurations, and effects of the present invention other than those described above will be clarified by the description of embodiments for carrying out the invention described below.

### Brief Description of Drawings

[FIG. 1] FIG. 1 is a schematic diagram illustrating a configuration of a prediction device according to one embodiment of the present invention.
[FIG. 2] FIG. 2 is a schematic diagram illustrating a configuration of an automatic cell culture system according to one embodiment of the present invention.
[FIG. 3] FIG. 3 is a flowchart of a method of controlling an automatic cell culture system according to one embodiment of the present invention.
[FIG. 4] FIG. 4 is a user interface of a prediction device system according to one embodiment of the present invention.
[FIG. 5] FIG. 5 shows a diagram showing a culture step in a step of inducing differentiation from iPS cells to dopamine neural progenitor cells, a graph showing expression levels of a marker gene of undifferentiated cells before the differentiation induction, and a graph showing expression levels of a marker of dopamine neural progenitor cells after the differentiation induction in Examples of the present invention.
[FIG. 6] FIG. 6 shows graphs showing changes in expression levels of miR-106a, miR-106b, miR-302a, miR-302b, miR-302c, and miR-302d per culture supernatant amount in the step of expansion culturing iPS cells and inducing differentiation from the iPS cells to dopamine neural progenitor cells in Examples of the present invention.

### Description of Embodiments

Hereinafter, various embodiments of the present invention will be described with reference to the drawings and Examples. However, it should be noted that these embodiments are merely examples for realizing the present invention, and do not limit the technical scope of the present invention. In the drawings, the same reference numerals are given to common configurations.

The present disclosure is based on the finding that, in a differentiation process of cultured cells (mainly having an expansion culture phase and a differentiation induction phase), measuring miRNAs contained in exosomes derived from cultured cells in a culture supernatant before or at the time of differentiation induction makes it possible to predict a differentiation level after the differentiation induction of the cultured cells is performed thereafter, i.e. a differentiation rate of the cultured cells to be terminally differentiated. Therefore, the present disclosure relates to a method and a device for predicting a differentiation level of such a cultured cell, as well as an automatic cell culture system using the device.

In one aspect, the present disclosure provides a prediction method for predicting a differentiation level of a cultured cell, including the steps of: measuring a content of miRNA in an exosome in a culture supernatant before differentiation induction of the cultured cell; and predicting the differentiation level of the cultured cell based on the content.

The type of the cultured cells to be predicted is not particularly limited as long as the cells are cells having the ability to differentiate by the differentiation induction process. Examples of the cells include pluripotent stem cells such as induced pluripotent stem (iPS) cells and embryonic stem (ES) cells, stem cells such as mesenchymal stem cells. Other human-derived cells and animal-derived cells, and established cultured cells or primary cultured cells may be used.

A direction of differentiation is also not particularly limited, and pluripotent stem cells include differentiation into all cell types, and differentiation into neural cells such as nerve cells and glial cells, visceral cells such as hepatocyte cells and pancreatic cells, blood cells such as erythrocytes and leucocytes, muscle cells such as skeletal muscle and cardiac muscle, immune cells such as T cells, B cells, and dendritic cells, epithelial cells, mucosal cells, stromal cells, and the like can be exemplified. In particular, differentiation into dopamine neural progenitor cells and dopaminergic neural cells are preferable. Therefore, in a preferred embodiment, the method of the present disclosure predicts the differentiation level of stem cells in the differentiation of stem cells into dopamine neural progenitor cells or dopaminergic neural cells.

The cultured cell can be subjected to expansion culture in a culture medium prior to the steps described above. Therefore, in one embodiment, the method of the present disclosure further includes a step of expansion culturing the cultured cell in a culture medium.

The "expansion culturing" refers to growing cells and increasing the number of cells. The culture medium to be used is not particularly limited as long as it is a medium suitable for such expansion culture, and can be appropriately selected depending on the type and number of cells to be used, the culture method, and the like. The period and conditions of expansion culture (such as temperature and carbon dioxide concentration) are also adjusted depending on the type of cells to be used, the direction of differentiation to be aimed, and the like. For example, when iPS cells are used, the expansion culture is performed at 30 to 40°C, for example, about 37°C, commonly for about 5 to 8 days, for example, for about 7 days. It should be noted that the terms "about" and "degree", as used herein together with numbers are meant to include ±10% of the numbers.

In the method of the present disclosure, the content of the miRNA in the exosome in the culture supernatant before differentiation induction of the cultured cell is measured. Here, the "before differentiation induction" refers to a time point after the above-described expansion culture and before the start of the differentiation induction process, or a time point between after the expansion culture and before the start of the differentiation induction process, for example, a time point from 3 days before differentiation induction to the start of differentiation induction, and may include immediately before the end of expansion culture or immediately after the start of the differentiation induction process in some cases. Specific determination before differentiation induction varies depending on the type of cells, the direction of differentiation, and the content of the differentiation induction process. The differentiation induction process also varies depending on the type of cells and the direction of differentiation, and can be performed, for example, by introducing a medium for promoting differentiation induction, adding a differentiation inducer to the medium, or the like.

In the present specification, the "culture supernatant" refers to a culture medium used for culture for a predetermined time. The culture medium at the start of culture is also included in the culture supernatant of the present disclosure. In the present specification, the component contained in the culture supernatant is also referred to as a culture supernatant component.

The component in the culture supernatant may be any component as long as it enables the differentiation level to be predicted. Examples of the component include RNA and proteins, i.e. exosomes or exosome markers, but are not limited thereto. The exosome marker is not particularly limited. For example, in the case of RNA, miRNA as one kind of RNA can be exemplified. Specifically, the miRNA may be at least one selected from the group consisting of miR-106 (miR-106a, miR-106b), miR-302 (miR-302a, miR-302b, miR-302c, and miR-302d). At least two combinations selected from the group consisting of miR-106a, miR-106b, miR-302a, miR-302b, miR-302c, and miR-302d, for example, a combination of one of miR-106a or miR-106b and one of miR-302a, miR-302b, miR-302c, or miR-302d may be measured. The sequence information of miR-106a, miR-106b, miR-302a, miR-302b, miR-302c, and miR-302d is known, miR-106a is registered as NCBI Gene ID: 406899 and miRBase: MI0000113, miR-106b is registered as NCBI Gene ID: 406900 and miRBase: MI0000734, miR-302a is registered as NCBI Gene ID: 407028 and miRBase: MI0000738, miR-302b is registered as NCBI Gene ID: 442894 and miRBase: MI0000772, miR-302c is registered as NCBI Gene ID: 442895 and miRBase: MI0000773, and miR-302d is registered as NCBI Gene ID: 442896 and miRBase: MI0000774, and are shown as SEQ ID NOs: 1 to 6 in the present specification.

The culture supernatant before differentiation induction of the cultured cell is collected, and the content of the miRNA in the exosome in the culture supernatant is measured. A method of detecting the miRNA in the exosome and a method of measuring the content of the miRNA are well known in the art, and are not particularly limited. Examples thereof include a method using probe hybridization (e.g. a method using an miRNA microarray or a Northern hybridization method), a method using a nucleic acid amplification technique (e.g. real-time PCR or digital PCR), a method using mass spectrometry, and a method using sequencing. The real-time PCR, the digital PCR, and the detection method using an miRNA array for detecting miRNA, with high sensitivity using a specific probe for the miRNA to be measured are preferable in terms of accuracy.

In the measurement of the content, the absolute amount or the relative amount of miRNA may be measured. It is preferable to obtain a measurement value suitable for comparison with a threshold described later.

The measurement of miRNA may be performed a plurality of times at other time points, for example, at the start of expansion culture, during expansion culture, after differentiation induction, or the like as long as the measurement is performed at least before differentiation induction. The change in the content of miRNA may be observed by performing the measurement a plurality of times.

In addition, a step of concentrating the exosome from the culture supernatant may be performed at a stage prior to the measurement of the content of miRNA in the exosome. The concentration method is not particularly limited, and for example, an ultracentrifugation method, a polymer precipitation method, a filter method, a surface antigen affinity method, a microdevice separation method, or the like can be used.

Subsequently, a step of predicting the differentiation level of the cultured cell based on the measured content of miRNA is performed. The "differentiation level of the cultured cell" refers to the number of cells terminally differentiated after the differentiation induction process with respect to the total number of cells subjected to the differentiation induction process, i.e. the differentiation rate of the cells. In the present invention, the future differentiation level of the cultured cell is predicted before the differentiation induction. Specifically, the differentiation level can be determined using the expression level or positive rate of a gene marker related to differentiation of the cultured cell as an index.

The prediction step can be performed, for example, based on a threshold derived from a correlation between a known differentiation level of a specific cultured cell and a content of miRNA. In one embodiment, such threshold information may be threshold information on the content of miRNA of the exosome based on the differentiation level of the cultured cell at the end of differentiation when the miRNA of the exosome is used as the target component. In another embodiment, the threshold information may be the content of miRNA of the exosome before the start of differentiation induction. A format of the threshold information is not particularly limited. It is preferable that the content of the component and the differentiation level of the cultured cell at the time point of the end of differentiation continuously correspond to each other. Examples of the format of the threshold information include calculation of a regression line or regression curve representing the relationship between the content of the component and the differentiation level of the cultured cell at the end of differentiation, or calculation of a threshold of the content of miRNA based on an equation representing either the regression line or the regression curve.

The threshold of the content of miRNA of the exosome based on the differentiation level of the cultured cell at the end of differentiation can be determined as follows.

The correlation between the content of miRNA in the exosome at a specific measurement time point and the differentiation level at the end of differentiation is determined. For example, it is conceivable to collect a culture supernatant at a predetermined time interval during expansion culture of cultured cells before differentiation induction and during the subsequent differentiation induction step, detect a marker (miRNA in exosome) in the culture supernatant to measure signal intensity, and prepare a regression line or regression curve representing the relationship between the signal intensity and the differentiation level at the end of differentiation. Here, it is also preferable to conduct experiments a plurality of times under the same differentiation conditions, take the average of the experiments, and create a regression line or regression curve representing the relationship between the signal intensity in the culture process and the differentiation level at the end of differentiation by the numerical value of the average.

Here, the method of determining the threshold of the content of miRNA of the exosome is not particularly limited. The threshold of the content of miRNA may be determined from the correlation information between the content of miRNA of the exosome and the undifferentiation level at the time of expansion culture before differentiation induction based on the correlation information between the undifferentiation level at the time of expansion culture before differentiation induction and the differentiation level at the end of differentiation culture of the cultured cells.

Since the threshold is the content of miRNA as a reference of a target differentiation level and varies depending on the target differentiation level, the threshold can be appropriately set depending on the type of cultured cells to be used, the expansion culture period, the measurement time of the content of miRNA, and the like.

In one embodiment, when the content of miRNA in the exosome measured before differentiation induction in the cultured cell is equal to or less than the threshold, it is predicted that the differentiation level of the cultured cell after differentiation induction satisfies the target differentiation level. Meanwhile, when the content of miRNA is higher than the threshold, it is predicted that the differentiation level of the cultured cell after differentiation induction does not reach the target differentiation level.

Alternatively, it is also possible to predict the differentiation level of the cultured cell after differentiation induction from the change in the content of miRNA in the exosome measured at a plurality of time points before, at the start of, and/or after the start of differentiation induction in the cultured cell.

In addition, cells under standard conditions having no problem in quality state are used as cells to be measured, miRNAs in exosomes in the respective culture supernatants of the cells to be measured and the cultured cells are measured before differentiation induction. Then, a ratio of the "content of miRNA in the culture supernatant of the cultured cells" with respect to the "content of miRNA in the culture supernatant of the cells to be measured" is determined, and when the determined value is equal to or less than a predetermined threshold, it may be predicted that the cultured cells satisfy the target differentiation level.

As described above, the differentiation level of the cultured cell can be predicted. Based on this prediction, it can be determined whether differentiation induction of the cultured cell is performed, and whether differentiation induction is continued after starting differentiation induction. Therefore, the method of the present disclosure may further include a step of determining whether differentiation induction of the cultured cell is performed based on prediction of the differentiation level of the cultured cell.

According to the method of the present disclosure, it is possible to predict cells whose differentiation induction efficiency will be reduced in the future at the start of differentiation induction. Thus, it is possible to omit a useless differentiation induction operation and to reduce the loss of cost and time. Further, the method of the present disclosure can noninvasively predict the differentiation level since miRNA: a component in the exosome released from the cultured cell is measured as a marker. Accordingly, the method is particularly useful, for example, when a closed system culture apparatus is used.

The method of the present disclosure as described above can be easily and simply performed by using a kit that includes a reagent, a container necessary for performing each step, an instruction including the description for performing the method (e.g. guidelines for prediction based on thresholds), and the like. Therefore, in another aspect, the present invention provides a kit for predicting a differentiation level of a cell, including a means (e.g., a primer and/or a probe) for measuring miRNA in an exosome derived from the cultured cell. The primer and/or the probe for measuring miRNA can be ordinarily designed by those skilled in the art based on the sequence information of miRNAs described above. The detection and measurement of miRNAs are ordinarily performed in the art, and kits for the detection and measurement of each of the miRNAs are commercially available. Thus, primers and/or probes included in such commercially available kits can also be used in the present kit.

For example, primers are designed to have a length and a base composition (melting temperature) that satisfy conditions allowing for specific annealing, for example, allowing for specific annealing. For example, the length with a function as a primer is preferably 10 bases or more, more preferably 15 to 30 bases, and still more preferably 15 to 20 bases. In designing the primer, it is preferable to confirm the GC content of the primer and the melting temperature (Tm) of the primer. Known primer design software can be used to confirm Tm. The designed primer can be chemically synthesized by a known oligonucleotide synthesis method, but is usually synthesized using a commercially available chemical synthesizer. In a specific embodiment, primers including at least 10 contiguous bases of the base sequences of the miRNA (SEQ ID NOs: 1 to 6) or their complementary sequences can be used singly, or in combination as a primer set.

For example, probes are designed to have a length and a base composition (melting temperature) that satisfy conditions allowing for specific hybridization, for example, allowing for specific hybridization. The length of a probe is preferably 10 bases or more, more preferably 20 to 50 bases, and still more preferably 20 to 30 bases. In a specific embodiment, probes including at least 10 contiguous bases of the base sequences of the miRNA (SEQ ID NOs: 1 to 6) or their complementary sequences can be used.

The primers and/or probes may include labels that are optimal for the detection method. Examples of such labels include, but are not limited to, radioisotopes (³²P, ¹²⁵I, ³⁵S, etc.), fluorescent substances (such as fluorescein (FITC), sulforhodamine (TR), and tetramethylrhodamine (TRITC)), and luminescent substances (such as luciferin).

When the kit includes a primer, the kit may further include a buffer constituting the reaction solution, a dNTP mixture, enzymes (such as reverse transcriptase), a standard sample for calibration, and the like. Alternatively, when the kit includes a probe, the kit may further include a hybridization buffer, a wash buffer, a microplate, a chip, and the like.

In addition, the method of the present disclosure can be easily and simply performed by using a device or system that includes a necessary device for performing each step. Hereinafter, a specific description will be given.

### == Prediction device for predicting differentiation level of cultured cell ==

In one aspect, the present invention provides a prediction device for predicting a differentiation level of a cultured cell, including an analysis device that is configured to predict the differentiation level of the cultured cell based on a content of miRNA in an exosome in a culture supernatant before differentiation induction of the cultured cell. The cultured cell, miRNA, and the like are as described above for the method of the present disclosure.

In one embodiment, the device of the present disclosure further includes a measurement device that is configured to measure a content of miRNA in the culture supernatant of the cultured cell. In one embodiment, the prediction device further includes a storage device, and the storage device stores correlation information between the content of the miRNA and the differentiation level after the differentiation of the cultured cell is completed, and a threshold of the content of the miRNA calculated from the correlation information.

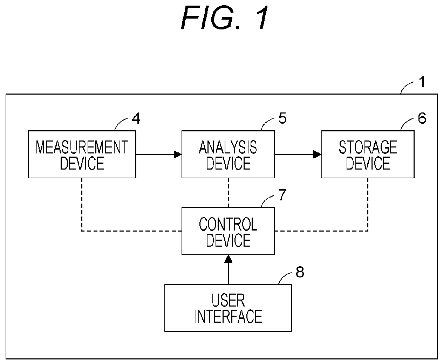

The prediction device for predicting a differentiation level of a cultured cell disclosed in the present specification includes an analysis device that is configured to predict the differentiation level of the cultured cell based on a content of miRNA in an exosome in a culture supernatant of the cultured cell (hereinafter, also simply referred to as "component" or "culture supernatant component"). Hereinafter, the prediction device of the present embodiment will be described in detail with reference to FIG. 1.

A prediction device 1 shown in FIG. 1 includes a measurement device 4 that is configured to measure a content of a component in a culture supernatant, an analysis device 5 that is configured to predict a differentiation level of a cultured cell based on the content, a storage device 6 that stores data obtained by analysis, a control device 7 that is configured to control the measurement device 4, the analysis device 5, and the storage device 6, and a user interface 8. The control device 7 is configured to control the prediction device 1. The user interface 8 inputs or outputs various types of information. Examples thereof include a keyboard, a mouse, a touch panel, a numeric keypad, a scanner, a microphone, a sensor, a display, a printer, and a speaker. Specifically, the user interface 8 may receive the measurement frequency of the culture supernatant component as an input, and outputs the measurement result of the culture supernatant component and the prediction result of the differentiation level. This device includes only one measurement device in FIG. 1, but may include a plurality of measurement devices. In this case, a plurality of samples can be simultaneously measured. These components may be connected through a wire or wirelessly.

The measurement device 4 includes an instrument capable of detecting a culture supernatant component, for example, exosomes or exosome markers and measuring the content of the exosomes or exosome markers, and may include, for example, a real-time PCR device, a digital PCR system, an miRNA array system, a spectrofluorometer, an ELISA reader, a stereomicroscope, a fluorescence microscope, or the like.

The storage device 6 is not particularly limited, but a non-volatile storage device is preferable, and examples thereof include a ROM, a flash memory, a magnetic storage device (such as hard disk drive, floppy disk, or magnetic tape), and an optical disk. The storage device 6 stores information including a threshold of the content of the component in the culture supernatant based on the differentiation level of the cultured cells at the end of differentiation. The threshold information is as described above, and may be, for example, correlation information between the content of miRNA of the exosome before the start of differentiation induction and the differentiation level of the cultured cells at the end of differentiation. A format of the threshold information is not particularly limited. It is preferable that the content of the component and the differentiation level of the cultured cell at the time point of the end of differentiation continuously correspond to each other. Examples of the format of the threshold information include calculation of a regression line or regression curve representing the relationship between the content of the component and the differentiation level of the cultured cells at the end of differentiation, or calculation of a threshold of the expression level of miRNA based on an equation representing either the regression line or the regression curve.

The analysis device 5 is not particularly limited, but may be, for example, a computer. The analysis device 5 is configured to predict the differentiation level of the cultured cell from the content of miRNA obtained by the measurement device 4 and the threshold information from the storage device 6. Alternatively, the analysis device 5 may be configured to predict the differentiation level of the cultured cell based on the change in the content of miRNA measured at a plurality of time points obtained by the measurement device 4. In one embodiment, the analysis device 5 is configured to predict the differentiation level of the cultured cell subjected to expansion culture for a certain period of time. In one embodiment, the analysis device may be configured to determine whether the culture of the cultured cell is continued and/or differentiation induction of the cultured cell is performed, based on the predicted differentiation level of the cultured cell.

### == Automatic cell culture system ==

In another aspect, the present invention provides an automatic cell culture system including:
a culture device including:
   a cell culture container for culturing a cell; and
   a drainage container for discarding a culture supernatant of the cultured cell; and
a prediction device including:
   a measurement device that is configured to measure a content of miRNA in an exosome in the culture supernatant before differentiation induction of the cultured cell; and
   an analysis device that is configured to predict a differentiation level of the cultured cell from the content of the miRNA. The cultured cell, miRNA, analysis device, measurement device, and the like are as described above for the method and device of the present disclosure.

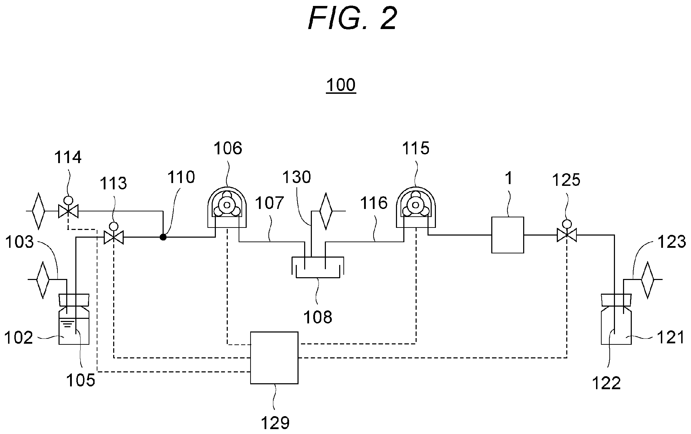

As illustrated in FIG. 2, the prediction device 1 may be connected to a culture device to form an automatic cell culture system 100 as a whole. The culture device is not particularly limited as long as it is a device capable of culturing cells. A device that has already been developed or will be developed in the future may be applied, and an example will be described below.

The automatic cell culture system 100 of the present disclosure has a first container 102 for containing a first liquid and a second container 108 for containing the first liquid. The first container 102 is a container for storing a culture medium for cell culture, i.e. the first liquid. The second container 108 is a container for cell culture, and the shape thereof is not particularly limited to a dish, a bottle, or the like, and is appropriately selected in accordance with the type of cells and the culture method. The first container 102 and the second container 108 can be easily produced by common technical knowledge of those skilled in the art in consideration of the respective purposes. Each has an air pressure adjustment tube 103 opened to the outside air, and has an end in a gas phase inside the container. The second container 108 has an air pressure adjustment tube 130 opened to the outside air, and has an end in a gas phase inside the container.

The culture device includes a liquid feeding tube 105 for feeding the first liquid in the first container 102 and a second liquid feeding tube 107 for feeding the first liquid in the first liquid feeding tube 105 to the second container 108. The second liquid feeding tube 107 includes a first liquid feeding pump 106 and adjusts feeding of the liquid in the second liquid feeding tube 107. Each of the liquid feeding tubes can be easily produced by common technical knowledge of those skilled in the art. The first liquid feeding tube 105 includes a first valve 113 and a second valve 114 which are connected through a connection portion 110, and opening and closing of each of the valves allows the presence or absence of liquid feeding to be switched.

Further, the culture device also has a third container 121 for discarding the first liquid in the second container 108. The third container 121 can be easily manufactured by common technical knowledge of those skilled in the art in consideration of the purpose. The third container 121 has an air pressure adjustment tube 123 opened to the outside air is provided, and has an end in a gas phase inside the container.

The culture device further includes a third liquid feeding tube 116 for discharging the first liquid in the second container 108, and a fourth liquid feeding tube 122 that is connected to the third liquid feeding tube 116 to discharge the first liquid in the second container 108 to the third container 121 through the third liquid feeding tube 116. The third liquid feeding tube 116 for carrying the culture supernatant to the third container 121 includes a second liquid feeding pump 115, and controls the liquid feeding in the third liquid feeding tube 116. Each of the liquid feeding tubes can be easily produced by common technical knowledge of those skilled in the art.

The culture device further has the third liquid feeding tube 116 connected from the second container 108 to the prediction device 1 and the fourth liquid feeding tube 122 connected from the prediction device 1 to the third container 121. The liquid feeding tube 122 has a third valve 125, and opening and closing allows the presence or absence of liquid feeding to be switched, and the measurement of the prediction device 1 can be started/stopped. As described above, the liquid feeding tube 122 has the third valve 125, and the opening and closing of the third valve 125 and ON and OFF of the prediction device 1 are adjusted in cooperation with each other.

The culture device may be provided with a control device 129 exclusively, and it is preferable that activation of the pumps and the opening and closing of the valves, and the like can be automatically controlled. The control device 129 may be connected to each component through a wire or wirelessly. Further, in the case of a culture device having a plurality of culture containers, the culture device preferably measures the content of the culture supernatant component for each of the containers.

In one embodiment, the prediction device 1 may configured to determine whether the culture device continues culture of the cultured cell and/or induces differentiation of the cultured cell based on the predicted differentiation level of the cultured cell, and then the control device 129 or the control device 7 is configured to control the culture device to continue or stop culturing the cultured cell and/or start differentiation induction based on the determination.

### == Method of operating automatic cell culture system ==

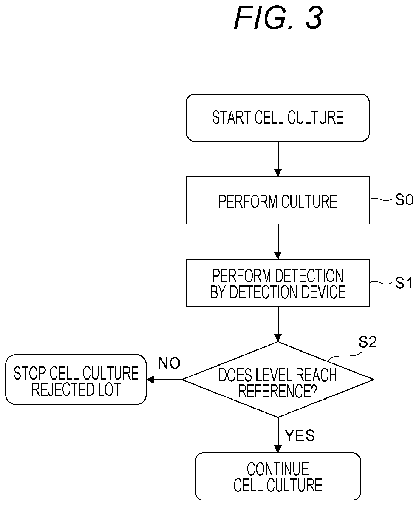

Hereinafter, a method of operating the automatic cell culture system 100 will be described in detail. The control of the automatic cell culture system 100 may be performed by a procedure or may be performed by the control device 7. FIG. 3 shows a flowchart when causing the control device 7 to perform the control.

First, the culture of cells to be measured is started, and the culture is continued (S0). After culturing the cells for a predetermined period, the content of the component in the culture supernatant is measured by the measurement device 4 using the methods as described above (S1). The analysis device 5 is configured to predict the differentiation level of the cultured cell at the end of differentiation using the threshold information of the content of the component and the differentiation level at the end of differentiation stored in the storage device 6 in advance.

When the predicted differentiation level has reached a predetermined reference value (S2), the cell culture is continued as it is. When the predicted differentiation level does not reach the predetermined reference value (S2), the control device 7 is configured to determine whether the culture is continued based on the prediction data of the differentiation level calculated by the analysis device 5 of the prediction device 1. For example, when the control device 7 is configured to determine that the culture is not continued, the control device 7 transmits the determination result to the automatic culture device within 12 hours, preferably within 6 hours, more preferably within 3 hours, and still more preferably within 1 hour after the calculation of the differentiation level, and stops the cell culture.

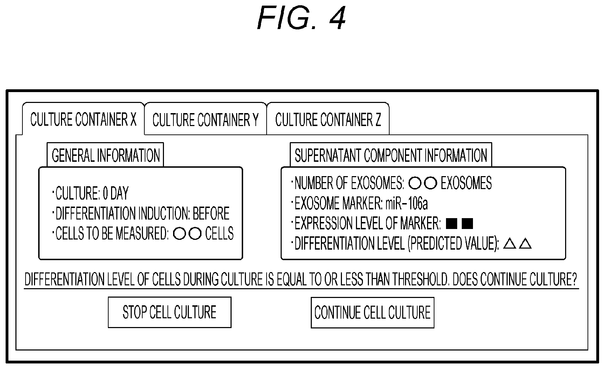

In addition, as illustrated in FIG. 4 as an example, the prediction data of the differentiation level may be output via the user interface 8. Based on the prediction data of the differentiation level, the presence or absence of future culture may be accepted as an input. Examples

### [Example 1]

This example shows that the expression level of exosome-derived miRNA in the culture supernatant before the start of differentiation induction changes depending on the undifferentiated state of iPS cells.

Specifically, differentiation induction was performed as follows (Doi, D. et al., Stem cell reports, 2.3, 337-350 (2014)). First, an iPS cell line 201B7 (Takahashi K, et al. Cell. 131 (5): 861-72 (2007)) was used to seed 1.3x10⁴ cells per well in a 6-well dish coated with LM511-E8 (E8 fragment of laminin 511). As culture conditions (deviation conditions) for deteriorating the quality of iPS cells, culture was performed without adding FGF2 necessary for maintaining the undifferentiated state from the 5th day after seeding. When the cells reached confluence after 7 days, a culture medium for growth (StemFit media) was replaced with a culture medium for differentiation (8% KSR, 0.1mM MEM, NEAA (all manufactured by Invitrogen), sodium pyruvate (Sigma-Aldrich), 0.1 mM 2-mercaptoethanol-containing GMEM). To promote neural differentiation, LDN193189 (STEMGENT) and A83-01 (Wako) were added. Further, to induce differentiation of floor plate cells, on the 1st to 7th days after the culture medium replacement, purmorphamine and FGF8 (Wako) were added, and on the 3rd to the 12th days, CHIR99021 (Wako/STEMGENT) was added. Thus, iPS cells were subjected to expansion culture for 7 days and induced to differentiate into dopamine neural progenitor cells for 12 days. The expression levels of 6 types of miRNAs were monitored by quantitative RT-PCR for the culture supernatants collected 6 days before the start of differentiation induction, 4 days before the start of differentiation induction, 2 days before the start of differentiation induction, 1 day before the start of differentiation induction, 0 day after the start of differentiation induction, 4 days after the start of differentiation induction, 8 days after the start of differentiation induction, and 12 days after the start of differentiation induction. Specifically, the expression levels were monitored using TaqMan Advanced miRNA Assays (Thermo Fisher Scientific, Inc., A25576) in accordance with the manufacturer's protocol. Primers 478623_mir, 478412_mir, 478006_mir, 478591_mir, 478509_mir, and 478237_mir of TaqMan Advanced miRNA Assays were used for miR-106a, miR-106b, miR-302a, miR-302b, miR-302c, and miR-302d, respectively.

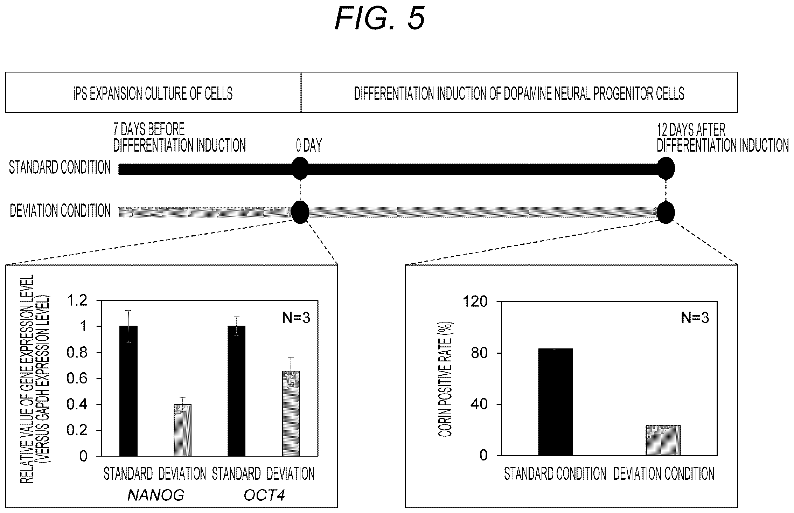

FIG. 5 shows a diagram showing the step of expansion culturing iPS cells and inducing differentiation of dopamine neural progenitor cells, expression levels of undifferentiated marker gene in the cultured cells before the start of differentiation induction, and a CORIN positive rate after differentiation induction. As is clear from the graph, in the deviation condition in which the expression levels of the undifferentiated marker gene was low, the CORIN positive rate after differentiation induction decreased as compared with the standard condition in which the expression level of the undifferentiated marker gene was maintained. CORIN is a marker for dopamine neural progenitor cells, and the CORIN positive rate indicates a differentiation induction efficiency.

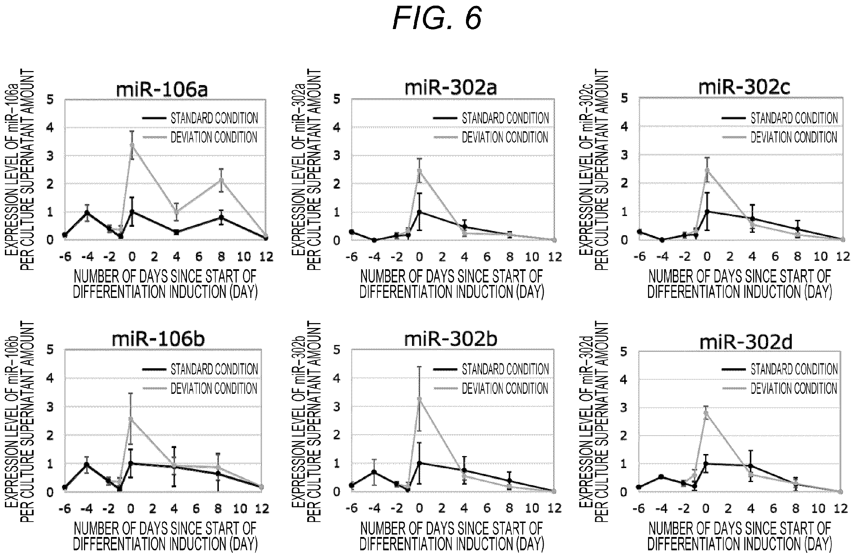

FIG. 6 shows changes in the expression levels of miR-106a, miR-106b, miR-302a, miR-302b, miR-302c, and miR-302d per culture supernatant amount in the step of expansion culturing iPS cells and inducing differentiation from the iPS cells to dopamine neural progenitor cells. The expression level of each of the miRNAs at the start of differentiation induction under standard conditions was 1, and the expression level of each of the other miRNAs was shown as a relative value.

As is clear from the graph, after 0 day from the start of differentiation induction, the expression levels of the 6 types of miRNAs under the deviation conditions were higher than those under the standard conditions.

As described above, the expression levels of the 6 types of miRNAs are monitored, and thus it is possible to predict cells whose differentiation induction efficiency will be reduced in the future at the start of the differentiation induction. This can greatly contribute to reduction in loss time and loss cost when a deviation in quality is found at the time of terminal differentiation.

All publications, patents, and patent applications cited herein are hereby incorporated by reference in their entirety.

### Reference Signs List

1 prediction device
4 measurement device
5 analysis device
6 storage device
7 control device
8 user interface
100 automatic cell culture system
102 first container
103 air pressure adjustment tube
105 first liquid feeding tube
106 first liquid feeding pump
107 second liquid feeding tube
108 second container
110 connection portion
113 first valve
114 second valve
115 second liquid feeding pump
116 third liquid feeding tube
121 third container
122 fourth liquid feeding tube
123 air pressure adjustment tube
125 third valve
129 control device
130 air pressure adjustment tube

## Claims

1. A prediction device for predicting a differentiation level of a cultured cell, comprising an analysis device that is configured to predict the differentiation level of the cultured cell based on a content of miRNA in an exosome in a culture supernatant before differentiation induction of the cultured cell.

2. The device according to claim 1, further comprising a measurement device that is configured to measure a content of miRNA in the culture supernatant of the cultured cell.

3. The device according to claim 1, wherein
the prediction device further includes a storage device, and
the storage device stores
correlation information between the content of the miRNA and the differentiation level after the differentiation of the cultured cell is completed, and
a threshold of the content of the miRNA calculated from the correlation information.

4. The device according to claim 1, wherein the analysis device is configured to predict the differentiation level of the cultured cell subjected to expansion culture for a certain period of time.

5. The device according to claim 1, wherein the miRNA is at least one selected from the group consisting of miR-106a, miR-106b, miR-302a, miR-302b, miR-302c, and miR-302d.

6. The device according to claim 1, wherein the cultured cell is a stem cell.

7. The device according to claim 6, wherein the stem cell is an iPS cell.

8. The device according to claim 6, wherein the analysis device is configured to predict a differentiation level of the stem cell in differentiation from the stem cell to a dopamine neural progenitor cell.

9. An automatic cell culture system comprising:
a culture device including:
a cell culture container for culturing a cell; and
a drainage container for discarding a culture supernatant of the cultured cell; and
a prediction device including:
a measurement device that is configured to measure a content of miRNA in an exosome in the culture supernatant before differentiation induction of the cultured cell; and
an analysis device that is configured to predict a differentiation level of the cultured cell from the content of the miRNA.

10. The system according to claim 9, wherein
the prediction device is configured to determine whether the culture device continues culture of the cultured cell and/or induces differentiation of the cultured cell based on the predicted differentiation level of the cultured cell, and
the system further includes a control device that is configured to control the culture device to continue or stop culturing the cultured cell and/or start differentiation induction based on the determination.

11. A prediction method for predicting a differentiation level of a cultured cell, comprising the steps of:
measuring a content of miRNA in an exosome in a culture supernatant before differentiation induction of the cultured cell; and
predicting the differentiation level of the cultured cell based on the content.

12. The method according to claim 11, further comprising a step of expansion culturing the cultured cell in a culture medium.

13. The method according to claim 11, further comprising a step of determining whether differentiation induction of the cultured cell is performed based on prediction of the differentiation level of the cultured cell.

14. The method according to claim 11, wherein the miRNA is at least one selected from the group consisting of miR-106a, miR-106b, miR-302a, miR-302b, miR-302c, and miR-302d.

15. The method according to claim 11, wherein the cultured cell is a stem cell.

16. Akit for predicting a differentiation level of a cultured cell, comprising a primer and/or a probe for measuring miRNA in an exosome derived from the cultured cell,
wherein the differentiation level of the cultured cell is predicted by a method including the steps of: measuring a content of miRNA in an exosome in a culture supernatant before differentiation induction of the cultured cell; and predicting the differentiation level of the cultured cell based on the content.
